(19) Europäisches Patentamt

European Patent Office

Office européen des brevets

(11) Veröffentlichungsnummer: **0 210 430**
**A2**

(12) # EUROPÄISCHE PATENTANMELDUNG

(21) Anmeldenummer: 86108523.1

(22) Anmeldetag: 23.06.86

(51) Int. Cl.⁴: **A61K 31/195**

Die Bezeichnung der Erfindung wurde geändert (Richtlinien für die Prüfung im EPA, A-III, 7.3).

(30) Priorität: 31.07.85 DE 3527356

(43) Veröffentlichungstag der Anmeldung:
04.02.87 Patentblatt 87/06

(84) Benannte Vertragsstaaten:
AT BE CH DE FR GB IT LI LU NL SE

(71) Anmelder: Degussa Aktiengesellschaft
Weissfrauenstrasse 9
D-6000 Frankfurt am Main 1(DE)

(72) Erfinder: Rüsse, Meinhard Werner, Prof. Dr.
Ginzing 3
D-8095 Schnaitsee(DE)
Erfinder: Bolze, Rudolf, Dr.
Grünaustrasse 11
D-6450 Hanau 9(DE)
Erfinder: Koch, Friedhelm, Dr.
Glatzer Strasse 22
D-6451 Grosskrotzenburg(DE)

(54) Verwendung von Tyrosin zur Verbesserung der Fruchtbarkeit von Säugetieren.

㊗ Die Fruchtbarkeit von weiblichen Säugetieren kann dadurch verbessert werden, daß man dem Tier einmalig oder innerhalb kurzer Zeit mehrmalig Tyrosin in einer hohen Dosis verabreicht. Die Verabreichnung kann wahlweise intravenös oder oral erfolgen.

EP 0 210 430 A2

## Mittel zur Verbesserung der Fruchtbarkeit von Säugetieren und Verfahren zu seiner Herstellung

Die Erfindung betrifft ein Mittel zur Verbesserung der Fruchtbarkeit von Säugetieren.

Das erfindungsgemäße Mittel ist insbesondere geeignet zur Verbesserung der Fruchtbarkeit von weiblichen geschlechtsreifen Säugetieren. Unter Verbesserung der Fruchtbarkeit ist dabei zu verstehen, daß z.B. bei weiblichen Tieren der Zyklus stimuliert und normalisiert wird. Die Verwendung des Mittels ist besonders bei landwirtschaftlichen Nutztieren, wie Rinder, Schafe, Pferde und Sauen, sowie bei Hunden und Katzen angezeigt.

Tyrosin gehört zu den in der Natur vorkommenden Aminosäuren und ist Bestandteil der Proteine. Es kann aus der essentiellen Aminosäure Phenylalanin gebildet werden und wird deshalb nur als semi-essentiell eingestuft. Tyrosin stellt den wichtigsten Metaboliten des Phenylalanins dar. Die Synthese findet in erster Linie in der Leber und in begrenztem Maße im Gehirn statt.

Neben ihrer Funktion als Bausteine im Proteinstoffwechsel haben Phenylalanin und Tyrosin auch noch Spezialaufgaben als Vorläufer von Hormonen zu erfüllen. So leiten sich unter anderem Dopamin, Noradrenalin, Adrenalin und Tyroxin vom Tyrosin und Phenylalanin ab.

Es wird vermutet, daß die Syntheserate von Tyrosin aus Phenylalanin oft nicht ausreicht, um den Tyrosinbedarf des Organismus zu jedem Zeitpunkt zu decken.

Es ist bekannt, daß Phenylalanin und Tyrosin, vorzugsweise jedoch bestimmte Derivate dieser Aminosäuren, zur Blutdruckregulierung und zur Appetitanregung verwendet werden können. Dem liegt die Beobachtung zugrunde, daß durch erhöhte Tyrosingaben die Katecholaminbiosynthese, vor allem die Synthese von Noradrenalin, stimuliert werden kann. Diese Neurotransmitter führen zu einer normalisierenden Regulation von Stoffwechsel-und physiologischen Erregungszuständen.

Der vorliegenden Erfindung liegt nun die überraschende Beobachtung zugrunde, daß durch einmalige oder innerhalb von vier Tagen mehrmalige, vorzugsweise 2-bis 4-malige, hohe Tyrosingaben das Fruchtbarkeitsgeschehen von Haustieren derart gesteuert werden kann, daß diese hohe Gabe von Tyrosin zu einer Zyklusstimulierung und -normalisierung führt.

Das erfindungsgemäße Mittel wird in der Weise angewandt, daß man dem Tier einmalig oder innerhalb kurzer Zeit mehrmalig Tyrosin in einer Menge zwischen 30 und 200 mg pro kg Körpergewicht,vorzugsweise zwischen 80 und 120 mg pro kg Körpergewicht, verabreicht. In der Tagesdosis sind dazu je nach Tierart 1 bis 50 g Tyrosin zu verabreichen.

Das Tyrosin kann beispielsweise in Form einer physiologisch verträglichen Lösung intravenös verabreicht werden, vorzugsweise in Form einer Lösung in steriler physiologischer Kochsalzlösung. Dazu besonders geeignet sind Tyrosin-Derivate, wie kurzkettige Peptide, besonders N-Glycyl-L-Tyrosin, N-Acetyl-L-Tyrosin und Alkylester des L-Tyrosins mit 1 bis 5 Kohlenstoffatomen, vorzugsweise die Methyl-und Ethylester, bzw. die Hydrochloride dieser Verbindungen, sowie Salze des Tyrosins, z.B. die Natrium-und Kaliumverbindungen.

Alternativ dazu kann auch das Tyrosin als solches, in Form seiner Kalium-, Natrium-, Magnesium-oder Calciumsalze oder in Form eines physiologisch verträglichen Derivates, aus dem im Verdauungstrakt Tyrosin freigesetzt wird, oral verabreicht werden. Geeignete Tyrosinderivate sind beispielsweise das N-Hydroxymethyl-Derivat, das N-Acetyl-und N-Glycyl-Derivat oder die N-Acyl-Derivate mit gerad-oder verzweigtkettigen, gesättigten oder ungesättigten Acylresten mit 8 bis 22 Kohlenstoffatomen.

Bei Wiederkäuern werden oral verabreichte Aminosäuren zum größten Teil in den Vormägen mikrobiell abgebaut. Soll daher Tyrosin in größerem Umfang im Stoffwechsel zur Verfügung stehen, muß es gegen diesen Abbau durch Mikroorganismen im Pansen geschützt werden. Für den Schutz der Aminosäure vor bakteriellem Abbau sind verschiedene Methoden geeignet. Einmal kann das Tyrosin chemisch derivatisiert werden. Derivate, die weitgehend unabgebaut durch den Pansen hindurchtreten, sind beispielsweise die bereits genannten N-Hydroxymethyl-und N-Acyl-Derivate. Zum anderen kann aber auch Tyrosin als solches in Form von gecoateten Teilchen verabreicht werden. Als Uberzugsmittel für das zu schützende Tyrosin können geeignete Polymere verwendet werden, besser aber dem Stoffwechsel unmittelbar zugängliche Substanzen, wie längerkettige Fettsäuren mit beispielsweise 14 bis 22 Kohlenstofatomen und Triglyceride. Ein besonders vorteilhaftes Überzugsmittel stellen Gemische aus freien Fettsäuren und den Natrium-, Kalium-, Calcium-oder Magnesiumsalzen der vorliegenden aliphatischen Monocarbonsäuren mit 14 bis 22 Kohlenstofatomen dar. Die Fettsäuren können gesättigt oder ungesättigt, verzweigt oder unverzweigt sein. Bevorzugt werden die unverzweigten, in natürlichen Fetten vorkommenden Fettsäuren, wie Myristinsäure, Palmitinsäure, Stearinsäure, Arachinsäure oder Behensäure oder Gemische solcher Säuren. Die Gemische können vorzugsweise aus ·50 bis 90 Gewichtsprozent Säure und 10 bis 50 Gewichtsprozent Salz bestehen. Es können auch hydrierte Fette mit Schmelzpunkten von 40 bis 60oC verwendet werden. Vorteilhafterweise wird deren Struktur durch geeignete Maßnahmen als Microteilchen fixiert, so daß sie der Resorption besser zugänglich sind. Bei oraler Verabreichung von Tyrosin, Tyrosin-Derivaten oder gecoateten Teilchen kann es zweckmäßig sein, zur Erleichterung und zum Sicherstellen einer vollständigen Aufnahme der beabsichtigten Tyrosinmenge das zu verabreichende Präparat zusätzlich in Gelatinekapseln einzuschließen. Die Tagesdosis kann dabei z.B. auf 2 oder 4 Weichgelatinekapseln verteilt werden, die gleichzeitig oder im Abstand von wenigen Stunden nacheinander mit einem Pillengeber verabreicht werden.

Beim Hund konnte beobachtet werden, daß nach Verabreichung einer einmaligen Tyrosingabe von 100 mg pro kg Körpergewicht die Belegung derart behandelter Hündinnen besonders erfolgreich verläuft. Das Tyrosin übt dabei einen deutlichen Einfluß auf den Zyklus aus, der weder aus den nutritiven noch aus den hormonellen Funktionen dieser Aminosäure bzw. ihrer Derivate erwartet werden kann.

Bei Muttersauen kann die Rausche nach dem Absetzen durch orale Tyrosingaben günstig beeinflußt werden. Sofern keine Rausche nach dem Absetzen eingetreten ist, kann diese auch nach längerer Zeit durch eine einmalige Tyrosingabe ausgelöst werden. Überraschend ist, daß bei den mit Tyrosin behandelten Sauen mit einer um 20 % gesteigerten Ferkelzahl pro Wurf gerechnet werden kann.

In weiteren Versuchen konnte der gleiche Effekt auch durch intravenöse Infusion eines physiologisch verträglichen leichtlöslichen Tyrosin-Derivates erzielt werden. Dabei wurde z.B. eine Lösung von 2 Gewichtsprozent N-Glycyl-L-Tyrosin in physiologischer Kochsalzlösung verwendet. Für Infusionslösungen werden vorzugsweise Lösungen mit 0,2 bis 5 Gewichtsprozent des Tyrosin-Derivats verwendet.

Kühe, denen eine Gabe von Tyrosin in einer mit Fettsäuren und Fettsäurealkalimetallsalzen geschützten Form verabreicht wurde, wurden nach 3 Tagen brünstig und konnten mit hoher Konzeptionsrate besamt werden. Für den Einsatz bei Milchkühen hat sich eine Mischung aus 20 bis 40 Gewichtsprozent des Überzugsgemisches und 60 bis 80 Gewichtsprozent Tyrosin als besonders vorteilhaft herausgestellt. Es können jedoch auch Mischungen mit niedrigeren Tyrosingehalten verwendet werden. Selbstverständlich können zusammen mit dem Tyrosin auch noch andere Nähr-oder Wirkstoffe, beispielsweise andere Aminosäuren oder Vitamine, geschützt und in dieser Form dem Wiederkäuer verabreicht werden.

Mittel für die orale Verabreichung können in der Dosierungseinheit 1 bis 10 000 mg, vorzugsweise 5 bis 4 000 mg, Tyrosin oder die dem Tyrosin äquivalente Menge eines Tyrosin-Derivates enthalten. Bei ihrer Herstellung können die üblichen Träger-und/oder Verdünnungs-und/oder Hilfsstoffe verwendet werden. Der Tyrosingehalt solcher Mittel kann im allgemeinen 50 bis 90 Gewichtsprozent betragen.

Vorzugsweise können die Mittel so hergestellt werden, daß man 50 bis 90 Gewichtsprozent Tyrosin oder die dem Tyrosin äquivalente Menge eines Tyrosin-Derivates zusammen mit üblichen Träger-und/oder Verdünnungs-und/oder sonstigen Hilfsstoffen bei einer Temperatur zwischen 20 und 60°C vermischt bzw. homogenisiert und die so erhaltene Mischung zur Herstellung eines Granulats, dessen einzelne Partikel 1 bis 200 mg Tyrosin enthalten, oder von Pillen, Pellets oder Tabletten, die 10 bis 500 mg Tyrosin enthalten, oder von Briketts, die 200 bis 10 000 mg Tyrosin enthalten, verwendet. Die Mischung kann alternativ auch in Kapseln aus Weichgelatine abgefüllt werden, die dann 200 bis 10 000 mg Tyrosin enthalten können.

Als Hilfsstoff bei der Herstellung des Mittels können beispielsweise Futtermittel, wie Weizenkleie, Stärke, mikrokristalline Cellulose oder Milchzucker, oder Calciumhydrogenphosphat verwendet werden, als Granulierhilfsmittel können beispielsweise Methylcellulosen, wäßrige Gelatinelösungen oder Stärkelösungen dienen.

Geeignete Mittel können auch in der Weise hergestellt werden, daß man 50 bis 90 Gewichtsprozent Tyrosin oder die dem Tyrosin äquivalente Menge eines Tyrosin-Derivates, gegebenenfalls nach Zusatz von Emulgatoren oder Fettsäureestern, z.B. Lecithin oder Monoester des 1,2-Propandiols mit Speisefettsäuren, bei einer Temperatur zwischen 40 und 60° C in geschmolzenem Hartfett oder in einer Mischung aus aliphatischen Monocarbonsäuren mit 14 bis 22 Kohlenstoffatomen und deren Natrium-, Kalium-, Calcium-oder Magnesiumsalzen suspendiert und homogenisiert und anschließend die homogenisierte Mischung in Hohlzellen ausgießt oder auf einer Pelletierpresse unter geeigneten Bedingungen Pellets oder Briketts preßt.

Die Erfindung wird durch die nachfolgenden Beispiele näher erläutert:

Beispiel 1:

Sechs deutsche Fleckviehkühe erhielten über einen Zeitraum von 4 Tagen L-Tyrosin in einer Dosierung von 4 g pro Tag als intravenöse Infusion in Form einer sterilen physiologischen Kochsalzlösung von N-Acetyl-L-Tyrosin verabreicht. Alle Versuchstiere wiesen nach der Geburt des ersten Kalbes eine Azyklie auf. Nach der Infusion stieg der Serumtyrosingehalt 1 bis 2 Tage vor der Ovulation stark an. Besamungen nach der Tyrosinbehandlung verliefen erfolgreich. Tiere, die nicht besamt wurden, wiesen danach wieder regelmäßige Zyklen auf.

Beispiel 2:

2 080 g einer Mischung von 1 700 g L-Tyrosin, 200 g Stearinsäure, 100 g Natriumstearat und 80 g Wasser wurden in einer Lochpresse agglomeriert und anschließend bei 65°C getrocknet. Das nach diesem Verfahren hergestellte Tyrosin-haltige Mittel wurde im Rahmen einer Feldstudie 19 Kühen, bei denen eine 50 Tage post partum andauernde Azyklie diagnostiziert worden war, in einer einmaligen Gabe von 40 g L-Tyrosin oral verabreicht. 3 bzw. 4 Tage nach der Tyrosinapplikation wurde bei sämtlichen Tieren mittels rektaler Untersuchung der Funktionszustand der Ovarien überprüft. In 17 Fällen wurden eindeutig sprungreife Follikel diagnostiziert.

Bei 18 Tieren waren Brunstsymptome deutlich erkennbar. Bei 17 besamten Tieren kam es in 11 Fällen zu einer Trächtigkeit. Vier weitere Tiere wurden 21 Tage später nachbesamt, von denen 2 konzipierten (insgesamt 80 % Konzeptionsrate).

Beispiel 3:

Zur Applikation entsprechend Beispiel 2 ist auch folgendes Mittel geeignet: 2 000 g einer Mischung von 1 200 g L-Tyrosin, 400 g eines gehärteten tierischen Fettes und 400 g Calciumstearat wurden unter Zugabe einer 0,5 gewichtsprozentigen Gelatinelösung bei 40 bis 50°C granuliert. Gegebenenfalls kann das Granulat auch in Gelatinekapseln gefüllt und mittels Pilleneingeber appliziert werden.

Beispiel 4:

In einem Ferkelerzeugerbetrieb wurde 16 Muttersauen 5 Tage nach dem Absetzen der Ferkel einmalig jeweils 15g L-Tyrosin mit dem Futter verabreicht, wobei ein L-Tyrosin-haltiges Mittel, hergestellt durch Verpressen unter Zuhilfenahme eines Presshilfsmittels einer Mischung von 47 Gewichtsprozent Weizengrießkleie, 3 Gewichtsprozent Melasse und 50 Gewichtsprozent L-Tyrosin, mit dem Sauenfutter vermischt wurde.

10 Sauen dienten als Kontrollgruppe. In der gleichen Weise wurde mit Erstlingssauen verfahren, wobei zwei Tiere als Kontrollgruppe dienten. Die Ergebnisse sind in der Tabelle aufgelistet.

| Altsauen | | Durchschnittliche Ferkelzahl |
|---|---|---|
| Versuchsgruppe | n = 16 | 11,8 |
| Kontrollgruppe | n. = 10 | 8,6 |
| Erstlingssauen | | |
| Versuchsgruppe | n = 3 | 11,3 |
| Kontrollgruppe | n = 2 | 7,0 |

Beispiel 5:

21 Hündinnen verschiedener Rassen erhielten am 5., 6. und 7. Tag des Prooestrus L-Tyrosin in einer Menge von jeweils 100 mg pro kg Körpergewicht. Hündinnen, die über 1 bis 2 Jahre nicht aufgenommen hatten, wurden nach der Tyrosingabe wieder trächtig. Die Symptome der Läufigkeit wurden nach der Applikation intensiviert und die Tiere ließen sich besser decken als in Läufigkeiten davor.

**Ansprüche**

1. Mittel, enthaltend als Wirkstoff Tyrosin oder ein Tyrosin-Derivat.

2. Mittel nach Anspruch 1 zur Verabreichung an geschlechtsreife weibliche Säugetiere, wie Milchkühe, Pferde, Schafe, Sauen, Hunde und Katzen, dadurch gekennzeichnet, daß es in der Dosierungseinheit 1 bis 10 000 mg, vorzugsweise 5 bis 4 000 mg, Tyrosin oder die dem Tyrosin äquivalente Menge eines Tyrosin-Derivates enthält.

3. Verfahren zur Herstellung eines Mittels nach Anspruch 2, dadurch gekennzeichnet,

daß man als Wirkstoff Tyrosin oder ein Tyrosin-Derivat zusammen mit üblichen Träger-und/oder Verdünnungs-und/oder Hilfsstoffen verwendet.

4. Verfahren zur Herstellung eines Mittels, dadurch gekennzeichnet,

daß man 50 bis 90 Gewichtsprozent Tyrosin, wobei das Tyrosin auch in Form eines Derivates vorliegen kann, zusammen mit üblichen Träger-und/oder Verdünnungs-und/oder Hilfsstoffen zu einem Mittel verarbeitet, welches in der Dosierungseinheit 1·bis 10 000 mg Tyrosin bzw. Tyrosin in Form eines Derivates enthält.

5. Verfahren zur Herstellung eines Mittels, dadurch gekennzeichnet,

daß man 50 bis 90 Gewichtsprozent Tyrosin, wobei das Tyrosin auch in Form eines Derivates vorliegen kann, zusammen mit üblichen Träger-und/oder Verdünnungs-und/oder Hilfsstoffen bei einer Temperatur zwischen 20 und 60° C vermischt bzw. homogenisiert und die so erhaltene Mischung zur Herstellung von Granulaten, die 1 bis 200 mg Tyrosin enthalten, Pillen, Pellets oder Tabletten, die 10 bis 500 mg Tyrosin enthalten, oder Briketts, die 200 bis 10 000 mg Tyrosin enthalten, benutzt.

6. Verfahren zur Herstellung eines Mittels, dadurch gekennzeichnet,

daß man 50 bis 90 Gewichtsprozent Tyrosin, wobei das Tyrosin auch in Form eines Derivats vorliegen kann, gegebenenfalls mit mindestens einem Futtermittel als Hilfsstoff, wie Weizenkleie, Stärke, mikrokristalline Cellulose, Calciumhydrogenphosphat oder Milchzucker vermischt oder unter Zusatz eines Granulierhilfsmittels, wie Methylcellulose oder einer wässrigen Gelatinelösung oder Stärkelösung granuliert oder zu Tabletten oder Pellets verpresst und/oder in Kapseln abfüllt.

7. Verfahren zur Herstellung eines Mittels, dadurch gekennzeichnet,

daß man 50 bis 90 Gewichtsprozent Tyrosin, wobei das Tyrosin auch in Form eines Derivats vorliegen kann, gegebenenfalls nach Zusatz von Emulgatoren oder Fettsäureestern (z.B. Lecithin oder Monoester des 1,2-Propandiols mit Speisefettsäuren) bei einer Temperatur zwischen 40 und 60° C in geschmolzenem Hartfett oder in einer Mischung aus aliphatischen Monocarbonsäuren mit 14 bis 22 Kohlenstoffatomen und deren Natrium-, Kalium-, Calcium-oder Magnesiumsalzen suspendiert und homogenisiert und anschließend die Mischung in Hohlzellen ausgießt oder auf einer Pelletpresse unter geeigneten Bedingungen Pellets oder Briketts presst.